# EUROPEAN PATENT SPECIFICATION

(11) **EP 3 836 918 B1**
(45) Date of publication and mention of the grant of the patent: **08.04.2026**
(21) Application number: 19755357.1
(22) Date of filing: 14.08.2019
(51) Int. Cl.: A61K 31/4015, A61K 31/5377, A61P 9/06

(54) **TASK-1 INHIBITORS FOR USE IN THE TREATMENT OF ATRIAL ARRHYTHMIAS**
TASK-1-INHIBITOREN ZUR VERWENDUNG IN DER BEHANDLUNG VON ATRIALER ARHYTHMIE
INHIBITEURS DES TASK-1 POUR L''UTILISATION DANS LE TRAITEMENT DE L'ARYTHMIE ATRIALE

(30) Priority: 15.08.2018 EP 18189182
(43) Date of publication of application: 23.06.2021
(73) Proprietor: Universität Heidelberg, 69117 Heidelberg (DE); Philipps-Universität Marburg, 35032 Marburg (DE)
(72) Inventor: SCHMIDT, Constanze, 69126 Heidelberg (DE); HAEFELI, Walter Emil, 69151 Neckargemünd (DE); KATUS, Hugo, 69120 Heidelberg (DE); DECHER, Niels, 35037 Marburg (DE); WIEDMANN, Felix, 69115 Heidelberg (DE); THOMAS, Dierk, 69120 Heidelberg (DE)
(74) Representative: Altmann Stößel Dick Patentanwälte PartG mbB
(86) International application number: PCT/EP2019/071858
(87) International publication number: WO 2020/035541

(56) References cited:
- US-A1- 2012 101 073
- US-A1- 2012 270 848
- COTTEN JOSEPH F ET AL: "The ventilatory stimulant doxapram inhibits TASK tandem pore (K2P) potassium channel function but does not affect minimum alveolar anesthetic concentration", ANESTHESIA AND ANALGE, WILLIAMS AND WILKINS, BALTIMORE, MD, US, vol. 102, no. 3, 1 January 2006 (2006-01-01), pages 779 - 785, XP009103929, ISSN: 0003-2999, DOI: 10.1213/01.ANE.0000194289.34345.63
- C. SCHMIDT ET AL: "BS54 - TASK-1 gene therapy suppresses atrial fibrillation in a large animal model", CLINICAL RESEARCH IN CARDIOLOGY, vol. 106, no. S2, 1 October 2017 (2017-10-01), Berlin/Heidelberg, pages 1 - 1, XP055545994, ISSN: 1861-0684, DOI: 10.1007/s00392-017-1154-6
- CONSTANZE SCHMIDT: "Upregulation of K2P3.1 K+ Current Causes Action Potential Shortening in Patients With Chronic Atrial Fibrillation", 1 May 2015 (2015-05-01), pages 82 - 92, XP055543517, Retrieved from the Internet <URL:https://www.ahajournals.org/doi/pdf/10.1161/CIRCULATIONAHA.114.012657> [retrieved on 20190117]
- JOSEPH F. COTTEN: "TASK-1 (KCNK3) and TASK-3 (KCNK9) Tandem Pore Potassium Channel Antagonists Stimulate Breathing in Isoflurane-Anesthetized Rats", ANESTHESIA & ANALGESIA, vol. 116, no. 4, 1 April 2013 (2013-04-01), pages 810 - 816, XP055213146, ISSN: 0003-2999, DOI: 10.1213/ANE.0b013e318284469d
- SHOTA OIKAWA ET AL: "Doxapram Hydrochloride Aggravates Adrenaline-Induced Arrhythmias Accompanied by Bidirectional Ventricular Tachycardia", ISRN CARDIOLOGY, vol. 2014, 1 January 2014 (2014-01-01), pages 1 - 5, XP055635829, DOI: 10.1155/2014/212045

## Description

The present invention relates to a compound having a structure as shown in formula (I) or a pharmaceutically acceptable salt thereof, 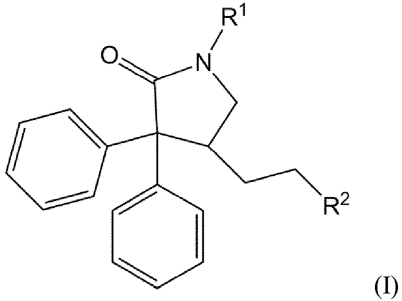
wherein R¹ is ethyl, -H, or methyl and R² is a side chain having a structure as shown in formula (II) 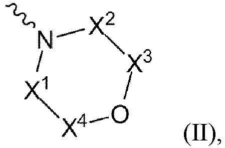
wherein X¹ is C=O, CH₂, C=S, C-OH, C-NH₂, or C-NO₂, X² to X⁴ are independently selected from CH₂, C=O, C=S, C-OH, and C-NH₂, or R² is -NH₂, for use in treating and/or preventing of atrial arrhythmia in a subject. The present invention further relates to pharmaceutical compositions related to said compound.

Atrial fibrillation (AF) is the most common cardiac arrhythmia. In the western world, 2 % of the population suffers from sustained AF, which is associated with an increased risk for heart failure, thromboembolic events, and mortality. In many cases, AF is first detected after a symptomatic stroke. Within the last years, hospitalization rates of patients with AF constantly increased. Due to demographic change, it can be expected that the number of AF patients will increase significantly over the next decades (Kirchhof et al. (2016), Eur Heart J 37:2893-962; Schmidt et al. (2011), Vasc Health Risk Manag 7:193-202; Singh et al. (2007), N Engl J Med 357:987-99; Kuck et al. (2016); N Engl J Med 374:2235-45).

The invasive and non-invasive approaches to revert AF that are currently available show low efficacy and high rates of AF recurrence. Current antiarrhythmic therapies are inefficient because of missing specificity, which is caused by an insufficient understanding of pathophysiological and molecular inductors of AF. At present, beta adrenoceptor inhibitors, flecainide, propafenone, digitalis glycosides, and amiodarone are used as pharmacologic treatment in AF patients. These antiarrhythmic drugs non-selectively target ion channels, which is in many cases linked to severe side effects for patients. Most of these drugs are multichannel inhibitors and therefore target ion channels not only in atrial but also in cardiac ventricular tissue, resulting in ventricular side effects and an increased risk of ventricular arrhythmias. During the last years, there was in intensive search for an atrium-selective target involved in AF induction. However, no therapeutic target could be identified that would currently enable atrium-specific therapy (Ravens & Odening (2017), Pharmacol Ther 176:13-21; Voigt & Dobrev (2016); Card Electrophysiol Clin 8:411-21; Ravens et al. (2013), J Physiol 591:4087-97).

In 2014, a new atrium-selective ion channel was discovered, the two-pore-domain potassium channel TASK-1 (TWIK-related acid-sensitive K(+)-1). TASK-1 inhibitors have been described e.g. in US 2015/0018342 A1. This channel showed a significant upregulation in patients with AF. Patch-clamp experiments in isolated human atrial cardiomyocytes from patients with AF showed that the TASK-1 channel contributed to the pathophysiologically typical shortening of atrial action potentials. Shortening of the atrial action potential in AF patients is an essential inductor and stabilizer of AF. Functional data showed that TASK-1 channel upregulation shortens the atrial action potential duration in patients with AF by about 30 % (Schmidt et al. (2015), Circulation 132:82-92; Schmidt et al. (2012), Dtsch Med Wochenschr 137:1654-8; Schmidt et al. (2014), Eur J Pharmacol 738:250-5; Schmidt et al. (2017), Eur Heart J 38:1764-74).

Schmidt et al. (2017), Clin Res Cardiol 106 Suppl 2, Abstract BS54 reported that TASK-1 gene therapy suppresses atrial fibrillation in a large animal model. Cotten et al. (2006), Anesth Analg 102:779 teaches Doxapram as inhibitor of TASK tandem pore (K₂ₚ) potassium channel function. US 2012/0270848 A1 relates to compositions and methods for treating a subject in need of opioid therapy, wherein the opioid therapy produces or has the possibility of producing respiratory depression or a breathing control disorder in the subject. US 2012/0101073 A1 relates to a method of treating a respiratory disease or disorder in a subject in need thereof, comprising administering to the subject a therapeutically effective amount of a pharmaceutical formulation comprising (+)-doxapram. Cotten (2013) AnesthAnalg. 116(4):. doi:l0.1213/ANE.Ob013e318284469d reported that TASK-1 and TASK-3 tandem pore potassium channel antagonists stimulate breathing in lsoflurane anesthetized rats. Oikawa et al. (2014), ISRN Cardiology 2014:1 teaches that Doxapram Hydrochloride aggravates Adrenaline induced arrhythmias accompanied by bidirectional ventricular tachycardia.

At present, doxapram (Dopram^{®}) is clinically indicated for drug-induced breathing suppression due to central nervous system (CNS) effects, for hypercapnia, for depressed breathing after general anaesthesia, and for neonates to enhance breathing.

There is, thus, a need in the art for providing improved means and methods for treating and preventing atrial arrhythmias, avoiding at least in part the drawbacks of the prior art. This problem is solved by the means and methods of the present invention, with the features of the independent claims. Preferred embodiments, which might be realized in an isolated fashion or in any arbitrary combination, are listed in the dependent claims.

Accordingly, the present invention relates to a compound having a structure as shown in formula (I) or a pharmaceutically acceptable salt thereof, 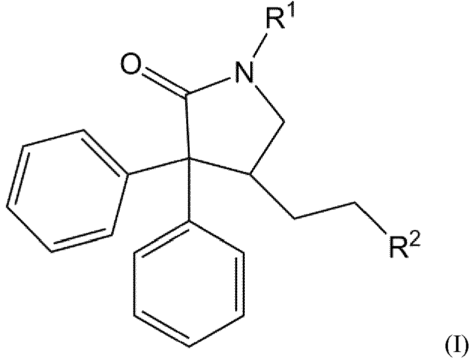 wherein
R¹ is ethyl, -H, or methyl and
R² is a side chain having a structure as shown in formula (II) 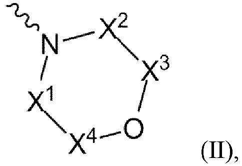 wherein
   X¹ is C=O, CH₂, C=S, C-OH, C-NH₂, or C-NO₂,
   X² to X⁴ are independently selected from CH₂, C=O, C=S, C-OH, and C-NH₂, or R² is -NH₂,
for use in treating and/or preventing of atrial arrhythmia in a subject.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements. Thus, preferably, the compound having a structure as shown in formula (I), preferably, consists of the indicated structure.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

As used herein, the term "standard conditions", if not otherwise noted, relates to IUPAC standard ambient temperature and pressure (SATP) conditions, i.e. preferably, a temperature of 25 °C and an absolute pressure of 100 kPa; also preferably, standard conditions include a pH of 7. Moreover, if not otherwise indicated, the term "about" relates to the indicated value with the commonly accepted technical precision in the relevant field, preferably relates to the indicated value ± 20 %, more preferably ± 10 %, most preferably ± 5 %. Further, the term "essentially" indicates that deviations having influence on the indicated result or use are absent, i.e. potential deviations do not cause the indicated result to deviate by more than ± 20 %, more preferably ± 10 %, most preferably ± 5 %. Thus, "consisting essentially of" means including the components specified but excluding other components except for materials present as impurities, unavoidable materials present as a result of processes used to provide the components, and components added for a purpose other than achieving the technical effect of the invention. For example, a composition defined using the phrase "consisting essentially of" encompasses any known acceptable additive, excipient, diluent, carrier, and the like. Preferably, a composition consisting essentially of a set of components will comprise less than 5 % by weight, more preferably less than 3 % by weight, even more preferably less than 1 %, most preferably less than 0.1 % by weight of non-specified component(s).

In the formulas provided, substituents (side chains), if not otherwise noted, have the usual meaning known to the skilled person. As is understood by the skilled person, a structure -X¹-with X¹ being C=O relates to a structure -C(O)-. Thus, preferably, the structure of formula (II) with X¹ being C=O and X² to X⁴ being CH₂ has the structure of formula (VII)

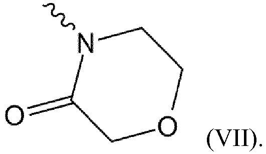

Preferably, at least two of X¹ to X⁴ are CH₂; more preferably, at least three of X¹ to X⁴ are CH₂, most preferably, X² to X³ are CH₂. Also preferably, X¹ and/or X² are not CH₂, more preferably, X¹ and/or X² is/are C=O, most preferably X¹ is C=O and X² is CH₂.

Preferably, the configuration at the stereocenter in position 4 of the 2-pyrrolidone-ring is the (R)-configuration. Thus, preferably, the compound of formula (I) has the structure of formula (Ia)

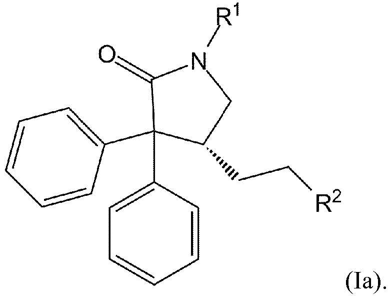

Preferably, R¹ is ethyl and X² to X⁴ are CH₂. More preferably, X¹ is C=O or CH₂, most preferably is C=O. Thus, preferably, the compound has the structure of formula (III), (IV), (V), or (VI)

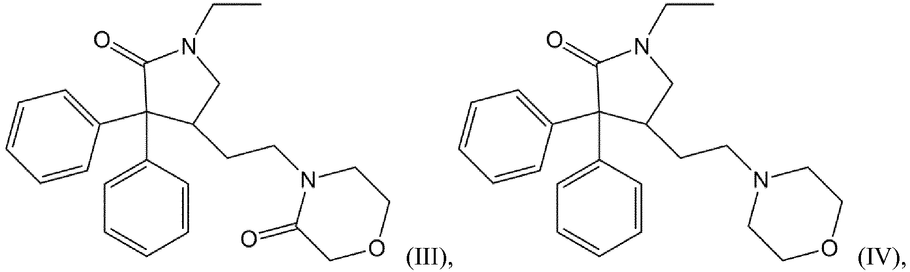

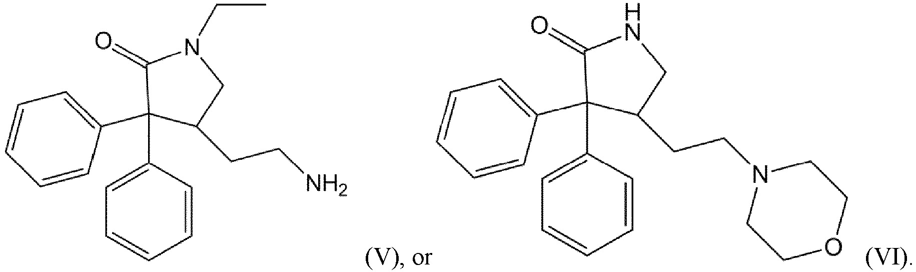

Preferably, the compound has the structure of formula (IV), i.e. is doxapram (1-ethyl-4-(2-morpholin-4-ylethyl)-3,3-diphenyl-pyrrolidin-2-one; CAS No: 309-29-5) or a pharmaceutically acceptable salt thereof. More preferably, the compound has the structure of formula (IV), i.e. is doxapram or a pharmaceutically acceptable salt thereof. Still more preferably, the compound has the structure of formula (IV), i.e. is doxapram or a hydrochloride thereof. Most preferably, doxapram is the racemic mixture of (R)- and (S)-doxapram, as marketed e.g. as Dopram^{®}. In a preferred embodiment, doxapram is (R)-doxapram, thus, in a preferred embodiment, doxapram is not (S)-doxapram ((-)-doxapram).

More preferably, the compound has the structure of formula (III), i.e. is 2-ketodoxapram (also referred to as "ketodoxapram" herein, 4-(2-(1-ethyl-5-oxo-4,4-diphenyl-3-pyrrolidinyl)ethyl)-3-morpholinone, CAS No: 42595-88-0) or a pharmaceutically acceptable salt thereof. Still more preferably, the compound has the structure of formula (III), i.e. is 2-ketodoxapram or a pharmaceutically acceptable salt thereof. Most preferably, the compound has the structure of formula (III), i.e. is 2-ketodoxapram or a hydrochloride thereof.

Still more preferably, the compound is (+)-2-ketodoxapram as described in WO 2012/166909 or a pharmaceutically acceptable salt thereof, i.e. preferably, 2-ketodoxapram has the (R)-configuration at the stereocenter referred to herein above, i.e. the compound preferably is (R)-2-ketodoxapram, preferably having a structure according to formula (IIIa)

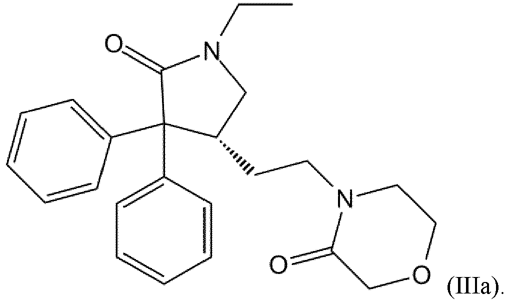

Still more preferably, the compound is (+)-2-ketodoxapram or a pharmaceutically acceptable salt thereof. Most preferably, the compound is (+)-2-ketodoxapram or a hydrochloride thereof. Preferably, (+)-2-ketodoxapram, as referred to herein, comprises at least 90 % of the (+)-enantiomer, more preferably at least 95 %, still more preferably at least 99 %, most preferably at least 99.9 % (+)-enantiomer. Methods for producing (R)-2-ketodoxapram (chiral resolution of a racemic mixture of (R)- and (S)-2-ketodoxapram or chiral synthesis via (S)-3-hydroxypyrrolidine) are reported in WO 2012/166909.

The term "pharmaceutically acceptable salt" is known to the skilled person and relates to any salt acceptable in the sense of not being deleterious to the recipient thereof and being compatible with optional other ingredients of a formulation of the pharmaceutically acceptable salt. Pharmaceutically acceptable salts are known in the art and comprise in particular chloride salts, in particular hydrochlorides, acetate salts, sulfate salts, and the like. Preferably, the pharmaceutically acceptable salt is a chloride salt, in particular a hydrochloride.

The term "treating" refers to an amelioration of the diseases or disorders referred to herein or the symptoms accompanied therewith, preferably to a significant extent. Said treating as used herein also includes an entire restoration of the health with respect to the diseases or disorders referred to herein. It is to be understood that treating as used in accordance with the present invention may not be effective in all subjects to be treated. However, the term shall require that, preferably, a statistically significant portion of subjects suffering from a disease or disorder referred to herein can be successfully treated. Whether a portion is statistically significant can be determined without further ado by the person skilled in the art using various well known statistic evaluation tools, e.g., determination of confidence intervals, p-value determination, Student's t-test, Mann-Whitney test etc.. Preferred confidence intervals are at least 90 %, at least 95 %, at least 97 %, at least 98 % or at least 99 %. The p-values are, preferably, 0.1, 0.05, 0.01, 0.005, or 0.0001. Preferably, the treatment shall be effective for at least 10 %, at least 20 %, at least 50 %, at least 60 %, at least 70 %, at least 80 %, or at least 90 % of the subjects of a given cohort or population. As will be understood by the skilled person, effectiveness of treatment of e.g. atrial arrhythmia is dependent on a variety of factors including, e.g. severity of disease, comorbidities, and the like. As is understood by the skilled person, as used herein, treatment preferably aims at reverting an atrial arrhythmia to a normal heart rhythm; thus, preferably, treating comprises or is cardioversion, more preferably chemical cardioversion.

The tem "preventing" refers to retaining health with respect to the diseases or disorders referred to herein for a certain period of time in a subject. It will be understood that the said period of time may be dependent on the amount of the drug compound which has been administered and individual factors of the subject discussed elsewhere in this specification. It is to be understood that prevention may not be effective in all subjects treated with the compound according to the present invention. However, the term requires that, preferably, a statistically significant portion of subjects of a cohort or population are effectively prevented from suffering from a disease or disorder referred to herein or its accompanying symptoms. Preferably, a cohort or population of subjects is envisaged in this context which normally, i.e. without preventive measures according to the present invention, would develop a disease or disorder as referred to herein. Whether a portion is statistically significant can be determined without further ado by the person skilled in the art using various well known statistic evaluation tools discussed elsewhere in this specification. Thus, preventing, preferably, is preventing occurrence of atrial arrhythmia and/or is preventing recurrence of atrial arrhythmia.

Preferably, the compound is not doxapram, more preferably the compound is 2-ketodoxapram as specified elsewhere herein and said use, i.e. in particular the treating and/or preventing as specified elsewhere herein, comprises avoiding adverse effects caused by central nervous stimulation, e.g. apprehension, disorientation, pupillary dilatation, hallucinations, headache, dizziness, hyperactivity, involuntary movements, muscle spasticity, muscle fasciculations, increased deep tendon reflexes, clonus, bilateral Babinski, nausea, vomiting, and/or convulsions; in particular avoiding epileptic seizures, paresthesias, restlessness, confusion, and/or hallucinations. Thus, preferably, the present invention relates to 2-ketodoxapram or a pharmaceutically acceptable salt thereof for treatment and/or prevention of a non-central nervous system-mediated disease or symptom, preferably atrial arrhythmia or chronic obstructive pulmonary disease associated with acute hypercapnia, more preferably atrial arrhythmia as specified elsewhere herein.

As used herein, the term "atrial arrhythmia" relates to any deviation from the normal rate or rhythm of one or both upper chambers of the heart. As understood by the skilled person, symptoms of atrial arrhythmia may include chest pain, fainting (syncope), palpitation, lightheadedness, and/or shortness of breath. As is understood by the skilled person, inhibition of TASK-1 will prolong the atrial action potential duration, which decreases the probability for induction or persistence of atrial arrhythmias including, preferably, atrial fibrillation, atrial flutter, premature atrial contraction, supraventricular tachycardias, sick sinus syndrome, and Wolff-Parkinson-White syndrome. Preferably, atrial arrhythmia is atrial fibrillation, atrial flutter, or a supraventricular tachycardia. Atrial fibrillation, preferably, is rapid and irregular beating of at least one atrium. Preferably, atrial fibrillation is characterized by the absence of P waves, presence of irregular electric activity in place of P-waves, and irregular P-P intervals in an ECG. Preferably, atrial fibrillation is paroxysmal atrial fibrillation, i.e. recurrent atrial fibrillation in which episodes stop within less than 7 days without treatment; or is persistent atrial fibrillation, i.e. recurrent atrial fibrillation in which episodes stop after more than 7 days without treatment; or is permanent atrial fibrillation, i.e. an ongoing long-term episode of atrial fibrillation. Also preferably, the atrial arrhythmia is atrial flutter, thus, preferably, is a sudden-onset, typically regular, abnormal heart rhythm on an electrocardiogram (ECG) in which the heart rate is fast.

The term "subject", as used herein, relates to a vertebrate animal, more preferably a mammal, in particular to livestock like cattle, horse, pig, sheep, and goat, or to a laboratory animal like a rat, mouse, and guinea pig. More preferably, the subject is a horse. Most preferably, the subject is a human.

Preferably, the use, i.e. in particular the treating and/or preventing as specified elsewhere herein, comprises avoiding concomitant use of an inhibitor or inducer of CYP3A4/3A5. The terms "CYP3A4" and "CYP3A5" are known to the skilled person to relate to two members of the CYP3A subfamily of cytochrome P450 monooxygenases. CYP3A4/3A5 catalyze the oxidation of doxapram to 2-ketodoxapram. Inhibitors and inducers of these enzymes are known in the art.

Also preferably, the use, i.e. in particular the treating and/or preventing as specified elsewhere herein, comprises administration of the compound, preferably as a pharmaceutical composition, at an appropriate dose, preferably an effective dose. The compound, preferably, is to be administered in combination with other drugs either in a common pharmaceutical composition or as separated pharmaceutical compositions wherein said separated pharmaceutical compositions may be provided in form of a kit of parts. Preferably, the pharmaceutical composition comprises both doxapram and 2-ketodoxapram in a single pharmaceutical composition. Preferably, the pharmaceutical composition further comprises at least one further cardioversion compound, i.e., preferably, an antiarrhythmic agent, and/or comprises at least one anticoagulant. Preferably, the further cardioversion compound is a beta blocker, preferably metoprolol, bisoprolol, or nebivolol; or a calcium channel blocker, preferably diltiazem or verapamil; or is a heart glycoside, preferably digoxin.

As used herein, the term "pharmaceutical composition", for which also the term "medicament" may be used, relates to a composition comprising at least the compound of the present invention and optionally one or more pharmaceutically acceptable carriers, i.e. excipient. The compound of the present invention can be formulated as pharmaceutically acceptable salts as specified elsewhere herein. The pharmaceutical composition is preferably prepared by combining the drug with standard pharmaceutical carriers according to conventional procedures. These procedures may involve mixing, granulating and compressing or dissolving the ingredients as appropriate to obtain the desired pharmaceutical composition. It will be appreciated that the form and character of the pharmaceutically acceptable carrier or diluent is dictated by the amount of active ingredient with which it is to be combined, the route of administration and other well-known variables. The excipient(s) must be acceptable in the sense of being compatible with the other ingredients of the formulation and being not deleterious to the recipient thereof. The excipient employed may be, for example, a solid, a gel or a liquid carrier. Exemplary of solid carriers are lactose, terra alba, sucrose, talc, gelatin, agar, pectin, acacia, magnesium stearate, stearic acid and the like. Exemplary of liquid carriers are phosphate buffered saline solution, syrup, oil such as peanut oil and olive oil, water, emulsions, various types of wetting agents, sterile solutions and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania. The diluent(s) is/are selected so as not to affect the biological activity of the compound. Examples of such diluents are distilled water, physiological saline, Ringer's solutions, dextrose solution, and Hank's solution. In addition, the pharmaceutical composition or formulation may also include other carriers, adjuvants, or nontoxic, nontherapeutic, non-immunogenic stabilizers and the like. Specific pharmaceutical compositions are prepared in a manner well known in the pharmaceutical art and comprise at least one active compound referred to herein above in admixture or otherwise associated with a pharmaceutically acceptable carrier or diluent. For making those specific pharmaceutical compositions, the active compound(s) will usually be mixed with a carrier or the diluent, or enclosed or encapsulated in a capsule, sachet, cachet, paper or other suitable containers or vehicles. The resulting formulations are to be adapted to the mode of administration, i.e. in the forms of tablets, capsules, suppositories, solutions, suspensions, sprays or powders for inhalation, patches, or the like and can have modified release and protective characteristics (e.g. slow release, enteric coating). Dosage recommendations shall be indicated in the prescribers or users instructions in order to anticipate dose adjustments depending on the considered recipient.

The compound or the pharmaceutical composition are, preferably, administered locally, topically or systemically, preferably are administered systemically. Suitable routes of administration of the compound or the pharmaceutical composition are oral, intravenous, intramuscular, subcutaneous, rectal, transdermal, or parenteral administration, as well as inhalation. Preferably, the compound or the pharmaceutical composition are, administered intravenously or orally. However, depending on the desired speed of action of a compound, the pharmaceutical composition may be administered by other routes as well.

A therapeutically effective dose refers to an amount of the compounds to be used in a pharmaceutical composition of the present invention which prevents, ameliorates or treats the symptoms accompanying a disease or condition referred to in this specification. Therapeutic efficacy and toxicity of such compounds can be determined by standard pharmaceutical procedures in cell cultures or experimental animals, e.g., ED₅₀ (the dose therapeutically effective in 50 % of the population) and LD₅₀ (the dose lethal to 50 % of the population). The dose ratio between therapeutic and toxic effects is the therapeutic index, and it can be expressed as the ratio, LD₅₀/ED₅₀. The dosage regimen will be determined by the attending physician based on clinical factors; preferably in accordance with any one of the above described methods. In view of the beneficial adverse events profile, it is, however, also envisaged that preferably doxapram, more preferably 2-ketodoxapram, are provided as self-medication preparations for self-administration e.g. in case an event of atrial arrhythmia occurs. As is well known in the medical arts, dosages for any one patient depends upon many factors, including disease severity and duration, success of previous therapies, the patient's size, body surface area, age, the particular compound to be administered, sex, genetics and genomics, differences in target expression (e.g. protein expression), time and route of administration, general health, food, co-morbidity, and other drugs being administered concurrently. Progress can be monitored by periodic assessment. A typical dose can be, for example, in the range of 0.1 to 1000 mg for a compound as referred to herein; however, doses below or above this exemplary range are envisioned, especially considering the aforementioned factors. The compound or pharmaceutical composition and formulations referred to herein are administered at least once in order to treat or ameliorate or prevent a disease or condition recited in this specification. However, the compound or pharmaceutical composition may be administered more than one time, for example from one to four times daily or continuously and up to a non-limited number of days. Preferably, the use, i.e. in particular the treating and/or preventing as specified elsewhere herein, comprises intravenous administration of said compound at a dose of from 0.1 mg/kg body weight to 10 mg/kg body weight, preferably of from 0.25 mg/kg body weight to 2 mg/kg body weight, more preferably of from 1.5 mg/kg body weight to 1.8 mg/kg body weight. Also preferably, the use comprises intravenous administration of said compound at a dose of from 0.004 g/day to 3 g/day, preferably of from 0.01 g/day to 0.3 g/day, more preferably of from 0.05 g/day to 0.27 g/day. More preferably, the use comprises oral administration of said compound at a dose of from 0.2 mg/kg body weight to 20 mg/kg body weight, preferably of from 0.5 mg/kg body weight to 4 mg/kg body weight, more preferably of from 3 mg/kg body weight to 3.6 mg/kg body weight. Also more preferably, the use comprises orally administering said compound at a dose of from 0.008 g/day to 6 g/day, preferably of from 0.02 g/day to 0.6 g/day, more preferably of from 0.1 g/day to 0.54 g/day. In a preferred embodiment, the standard time period between administration of doses is of from 8 hours to 48 hours, preferably of from 12 hours to 24 hours.

Advantageously, it was found that doxapram is effective in cardioversion of atrial arrhythmias, in particular atrial fibrillation. Surprisingly, it was found that a natural metabolite of doxapram, i.e. 2-ketodoxapram, has an efficacy in cardioversion comparable to doxapram, while passing the blood-brain barrier to a much lower extent. Thus, it was found that central nervous adverse effects of doxapram can be reduced or avoided altogether by using 2-ketodoxapram, while maintaining efficacy in cardioversion. Moreover, it was found that the ion channel Kv1.5, which is inhibited by conventional multi-channel blockers, is not inhibited by doxapram or ketodoxapram. Therefore, side effects of other antiarrhythmic drugs due to inhibition of Kv1.5, such as pulmonary hypertension or pro-arrhythmic effects, will not be caused by doxapram or ketodoxapram.

The definitions made above apply mutatis mutandis to the following. Additional definitions and explanations made further below also apply for all embodiments described in this specification mutatis mutandis.

The present invention further relates to a method for treating and/or preventing atrial arrhythmia in a subject comprising contacting said subject with an effective dose of a compound of the present invention, thereby treating and/or preventing atrial arrhythmia.

The method of the present invention, preferably, is an in vivo method. Moreover, it may comprise steps in addition to those explicitly mentioned above. For example, further steps may relate, e.g., to diagnosing atrial arrhythmia before contacting said subject with a compound of the present invention and/or providing further treatment to said subject, e.g. electrical cardioversion, additional medication, hospitalization, or the like. Moreover, one or more of said steps may be performed by automated equipment.

The present invention also relates to a use of a compound of the present invention for the manufacture of a medicament for treating and/or preventing atrial arrhythmia.

The present invention further relates to a pharmaceutical composition comprising at least one compound of the present invention for use in treating and/or preventing atrial arrhythmia in a subject.

The present disclosure also relates to a kit comprising at least one compound as specified in any one of claims 1 to 4 and a cardioversion device, and/or a further cardioversion medicament.

The term "kit", as used herein, refers to a collection of the aforementioned components. Preferably, said components are combined with additional components, preferably within an outer container. The outer container, also preferably, comprises instructions for carrying out a method of the present invention. Examples for such components of the kit as well as methods for their use have been given in this specification. The kit, preferably, contains the aforementioned components in a ready-to-use formulation. Preferably, the kit may additionally comprise instructions, e.g., a user's manual for applying the compounds with respect to the applications provided by the methods of the present invention. Details are to be found elsewhere in this specification. Additionally, such user's manual may provide instructions about correctly using the components of the kit. A user's manual may be provided in paper or electronic form, e.g., stored on CD or CD ROM. The present invention also relates to the use of said kit in any of the methods according to the present invention.

The term "cardioversion device" is known to the skilled person and relates to a device having essentially the functionality of a defibrillator and, optionally, an electrocardiogram device. Preferred further cardioversion compounds have been described herein above; as is understood by the skilled person, in case a further cardioversion compound is comprised in a kit as specified herein, the same further cardioversion compound is preferable not combined with the compound as specified herein as a pharmaceutical composition in the kit.

### Figure Legends

Fig. 1: Inhibitory effects of doxapram and ketodoxapram on TASK-1 K-ion channels, recorded by voltage-clamp measurements in Xenopus laevis oocytes. A) Concentration-effect curve for doxapram on porcine TASK-1; B) Concentration-effect curve for doxapram on human TASK-1; C) Concentration-effect curve for ketodoxapram on human TASK-1; D) % inhibition of ion channels TASK-1 (K2P3.1), TASK-3 (K2P9.1), Kv4.3, Kv1.5, Kv2.1, Kir3.1/4, and Kv1.4 by doxapram (black bars) and ketodoxapram (white bars), respectively. E) Inhibition of TASK-1 by doxapram, ketodoxapram, and mixtures thereof.
Fig. 2: Effects of ketodoxapram and doxapram in human atrial cardiomyocytes recorded by voltage-clamp measurements.
Fig. 3: Pharmacokinetics of doxapram and ketodoxapram in pigs after intravenous administration. Concentration of doxapram and ketodoxapram, respectively, as measured in plasma samples taken at the indicated time points.
Fig. 4: : Quantification of doxapram and ketodoxapram in porcine tissue samples. A) doxapram and ketodoxapram were quantified in brain tissue samples (CNS) and plasma samples of two pigs; B) Concentrations of doxapram and ketodoxapram in brain tissues of pigs treated with doxapram, which was metabolized to ketodoxapram.
Fig. 5: Action potentials measured in human atrial cardiomyocytes isolated from heart auricles of patients with sinus rhythm (A) or with chronic atrial fibrillation (B, C). The action potential could be reduced and prolonged by doxapram treatment (C).
Fig. 6: Correlation between plasma concentration and brain concentration of doxapram and its metabolite ketodoxapram in pigs after intravenous administration of doxapram; pig numbers and their group status are indicated (SR: sinus rhythm; AF: atrial fibrillation).
Fig. 7: Correlation between plasma concentration and brain concentration of ketodoxapram in pigs after intravenous administration of ketodoxapram; pig numbers and their group status are indicated (AF: atrial fibrillation).
Fig. 8: Short-term distribution of doxapram and its metabolite ketodoxapram in pigs 30 min after administration of doxapram; pig numbers and their group status are indicated (SR: sinus rhythm).
Fig. 9: Plasma trough concentrations of doxapram and its metabolite ketodoxapram in pigs during repetitive intravenous administration of doxapram over 18 days.
Fig. 10: Plasma concentrations of ketodoxapram in pigs during long-term administration of ketodoxapram.
Fig. 11: Pharmacokinetics of doxapram and its metabolite ketodoxapram in a horse after three intravenous administrations of doxapram.

The following examples shall merely illustrate the invention. They shall not be construed, whatsoever, to limit the scope of the invention.

### Example 1

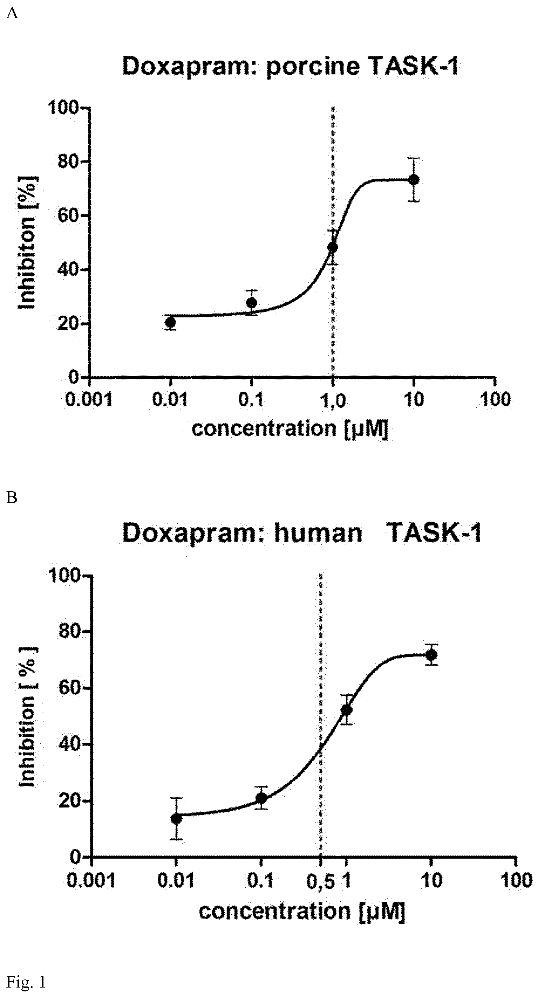

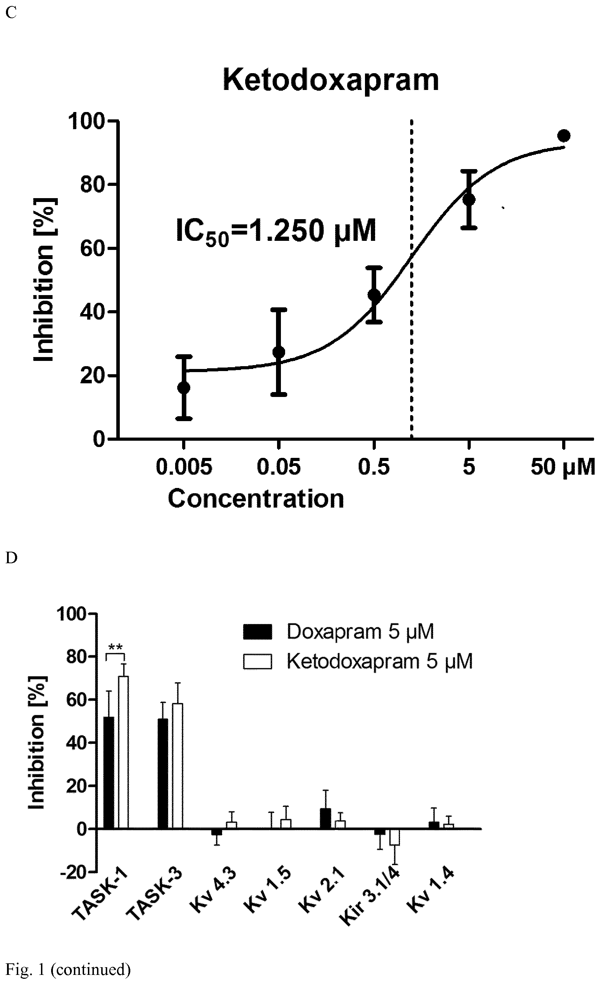

Concentration-response curves for ketodoxapram and doxapram. TASK-1 current inhibition was recorded by voltage clamp measurements in Xenopus laevis oocytes according to standard protocols. An IC₅₀ value of 1 µM was determined for doxapram inhibition of porcine TASK-1 (Fig. 1A)); of 0.5 µM for doxapram inhibition of human TASK-1 (Fig. 1B)), and of 1.25 µM for ketodoxapram inhibition of TASK-1 (Fig. 1C)).

The ion channels TASK-1 (K2P3.1), Kv4.3, Kv1.5, Kv2.1, Kir3.1/4, Kv1.4 are predominantly expressed in the atrial myocardium. The ion channel TASK-3 (K2P9.1) is not expressed in the human myocardium but structurally related to TASK-1 (Schmidt C et al. (2015), Circulation 132(2): 82). Ion channel current inhibition was measured in Xenopus laevis oocytes (Fig. 1 D)). TASK-1 was the most strongly inhibited ion channel. The effect of ketodoxapram on TASK-1 was significantly stronger than the effect of doxapram.

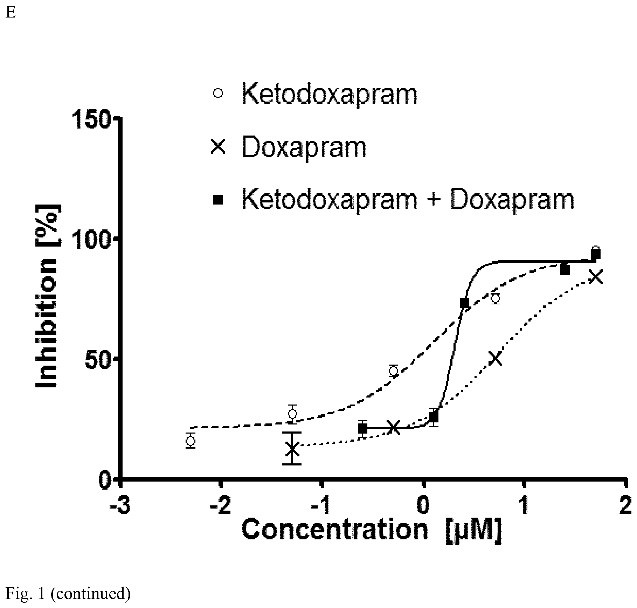

To test for competition between ketodoxapram and doxapram, concentration-response curves were determined by measuring TASK-1 current inhibition recorded by voltage clamp measurements in Xenopus laevis oocytes. As shown in Fig. 1E), if both compounds are present, doxapram is replaced at the ion channel by ketodoxapram due to its stronger affinity to TASK-1.

### Example 2

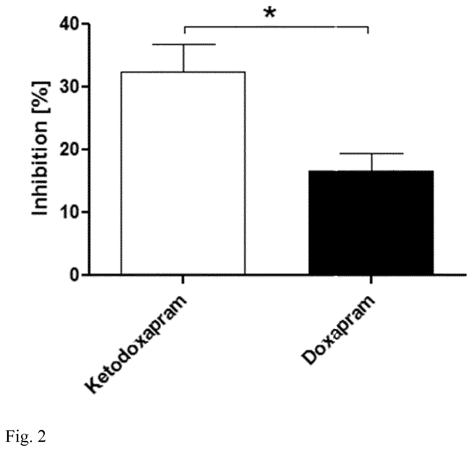

Effects of ketodoxapram and doxapram in human atrial cardiomyocytes: Experimental measurements of TASK-1 current inhibition recorded by patch-clamp measurements in human atrial cardiomyocytes isolated from heart auricles of patients with sinus rhythm (Fig. 2) showed that TASK-1 inhibition by ketodoxapram was significantly stronger than inhibition by doxapram. Since it is known that TASK-1 levels are significantly increased in patients with atrial fibrillation (Schmidt et al. (2015), Circulation 132(2): 82, Schmidt et al. (2017), European Heart Journal 38(22):1764) TASK-1 inhibition by ketodoxapram and doxapram will be even more efficient in patients with atrial fibrillation than in patients with sinus rhythm.

### Example 3

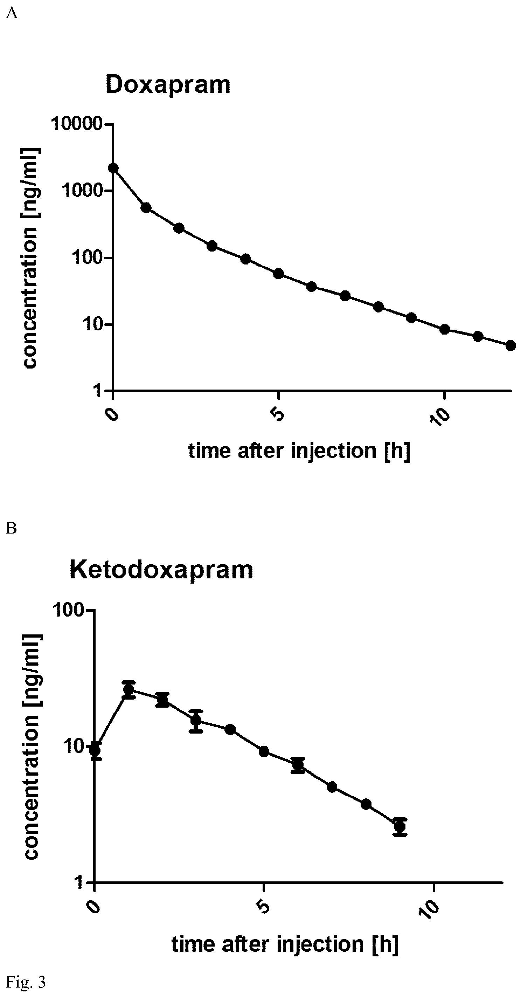

Pharmacokinetics of doxapram and ketodoxapram. Pigs were treated with doxapram (1.8 mg/kg body weight); thereafter, plasma samples were taken to quantify concentrations of doxapram (Fig. 3A) and ketodoxapram (Fig. 3B), indicating that doxapram was rapidly metabolized to ketodoxapram. Notably, upon oral administration of enteric-coated doxapram, the concentrations of doxapram and ketodoxapram in plasma are about equal (Robson & Prescott (1978), Br. J. Pharmac. 7:81; "AHR 5955" is ketodoxapram).

### Example 4

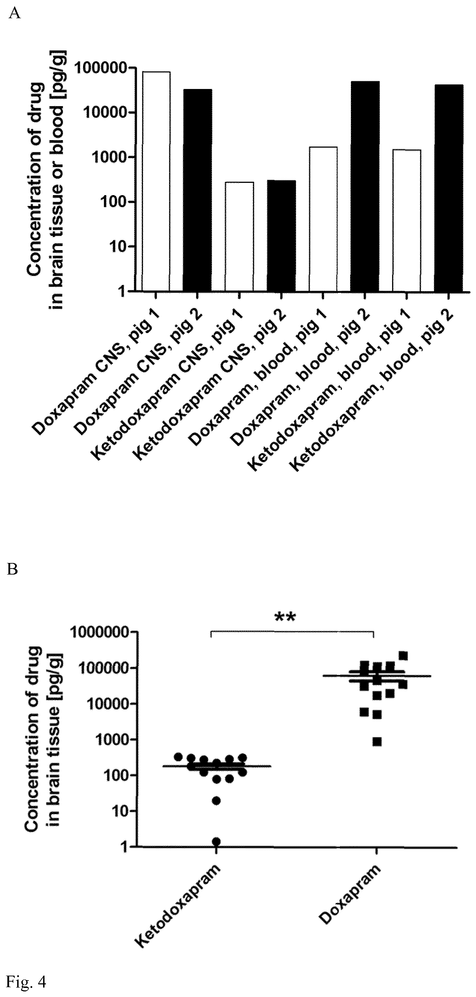

Quantification of ketodoxapram and doxapram in porcine tissues. In two pigs, doxapram and ketodoxapram were administered and quantified in brain tissue samples (CNS) and plasma samples (Fig. 4A). Data from the two pigs shown in Fig. 4A indicate that plasma concentrations of doxapram and ketodoxapram were similar at the time of analysis. Doxapram concentrations in the brain tissue samples were similar to the plasma concentrations, confirming earlier observations in dogs (Bruce et al. (1965), J Med Chem 8:157). In contrast, the concentrations of ketodoxapram in brain tissue samples were several orders of magnitude lower than the concentrations of doxapram in the same tissue.

When pigs were treated with doxapram, the compound was metabolized to ketodoxapram. The concentrations of the two compounds were determined in brain tissue, finding that the concentration of ketodoxapram is several orders of magnitude lower (Fig. 4B).

### Example 5

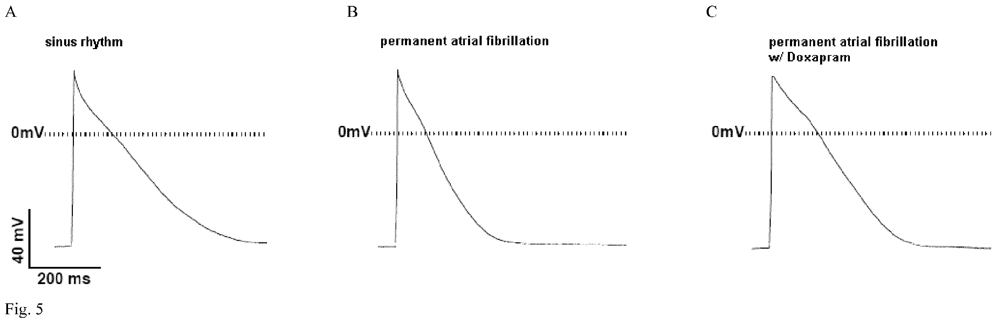

Action potentials measured in human atrial cardiomyocytes. Action potentials were recorded in atrial cardiomyocytes isolated from heart auricles of patients with sinus rhythm (Fig. 5A) or with chronic atrial fibrillation (Fig. 5B, C). In chronic atrial fibrillation, the action potential was reduced and could be prolonged by doxapram treatment (Fig. 5C). After doxapram treatment, the action potential duration was similar to sinus rhythm.

### Example 6

Synthesis of ketodoxapram. Ketodoxapram was synthesized essentially according to the protocol proposed in Example 1 of WO 2012/166909.

### Example 7

Analysis of doxapram and ketodoxapram in tissues. Mass spectrometry, in particular liquid chromatography tandem-mass spectrometry (LC-MS/MS) with deuterated internal standard, was used to quantify the concentrations of doxapram and ketodoxapram in tissue samples and plasma.

### Example 8

Adverse effects observed in pigs. Pigs (40 - 60 kg body weight) were treated with doxapram or ketodoxapram, respectively. In case of overdosing, animals treated with doxapram developed clear signs of central nervous adverse effects (increased breathing impulse, hyperventilation, nausea, agitation, restlessness, and trembling), which resolved over several minutes. These effects were not observed when an overdose of ketodoxapram was administered.

### Example 9

Doxapram and ketodoxapram were successfully applied for cardioversion in pigs. To induce atrial fibrillation in pigs, at first, pacemakers were implanted. Atrial fibrillation was induced by atrial rapid pacing. After 21 days, persistent atrial fibrillation was induced. doxapram was intravenously applied at a dose of 1.8mg per kg body weight, and ketodoxapram was intravenously applied at a dose of 0.5mg per kg body weight. These dosages were sufficient for cardioversion in all tested animals (n = 20). On average, pharmacological cardioversion to sinus rhythm was successful 3 minutes after injection of doxapram or ketodoxapram.

### Example 10

For cardioversion of atrial fibrillation (AF) in humans, doxapram can be injected via a peripheral venous catheter. The dose will be adapted to the body weight of the patient. Cardioversion is achieved within a few minutes (< 30 minutes). The ECG of the patient is continuously monitored to detect the presence of atrial fibrillation (AF) or successful cardioversion. In case of an unsuccessful cardioversion, a second dose can be applied. If AF is present after applying the second dose, a conventional electrical cardioversion can be performed. To prevent AF episodes, patients can receive repeated a maintenance doses adapted to the body weight for oral or intravenous application.

To treat atrial fibrillation outside of medical institutions such as a hospital or a cardiological practice, it is possible to orally administer doxapram or ketodoxapram, optionally be self-medication. Due to differences in bioavailability, the dose required for oral intake will be about two-fold higher than for intravenous application.

### Example 11: Intravenous pharmacokinetics of doxapram and ketodoxapram in pig and horse

### a) Distribution after intravenous doxapram administration

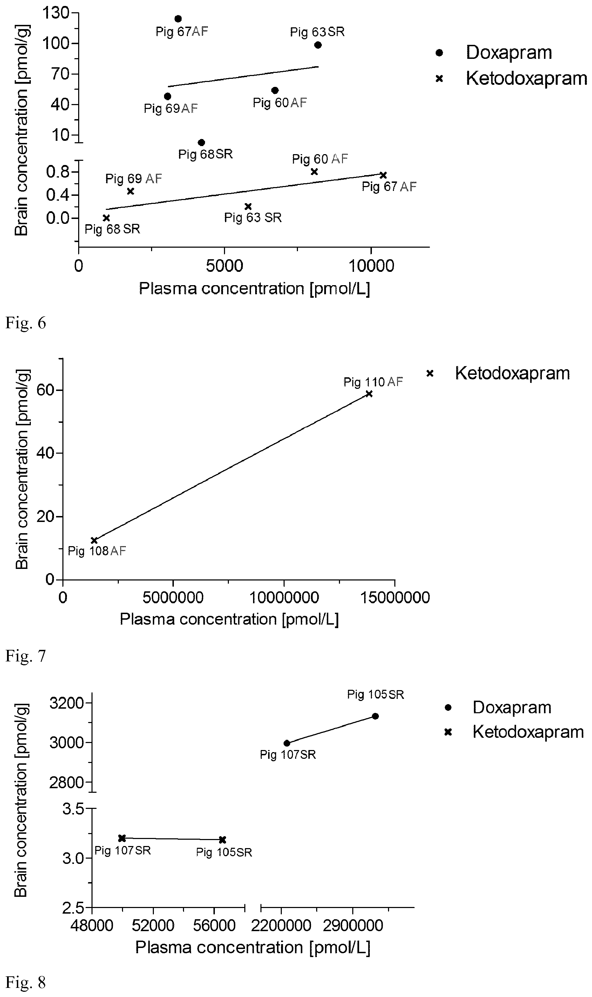

Pigs with sinus rhythm or atrial fibrillation were treated with intravenous doxapram and plasma and brain samples were taken essentially simultaneously. As is shown in Fig. 6, doxapram accumulates in the brain 60-500 times more extensively than ketodoxapram. Thus, the ketodoxapram concentration in the brain is only 0.2 to 1.7 % of the corresponding doxapram concentration. Fig. 6 also reveals that the brain concentration of doxapram is 0.24 % of its plasma concentration, whereas the corresponding value for ketodoxapram is only 0.0016 %, i.e. by factor 156 lower.

### b) Distribution after intravenous administration of ketodoxapram

After intravenous administration of ketodoxapram at a dose comparable to the doxapram dose used in a), the brain concentration of ketodoxapram is higher (Fig. 7), but nonetheless is only about 0.0006 % of the plasma concentration. Doxapram was not detected after ketodoxapram administration.

In conclusion, ketodoxapram crosses the blood-brain barrier much less effectively than doxapram.

### c) Initial distribution of doxapram and its active metabolite ketodoxapram

In two pigs, doxapram and ketodoxapram concentrations were determined in brain and plasma 30 min after intravenous administration. As shown in Fig. 8, doxapram brain concentrations are 0.1 % of the plasma concentration already after this time, i.e., distribution is rapid, quickly reaching similar brain:plasma ratios as observed after long-term exposure (cf. above).

### d) Initial distribution after intravenous doxapram administration

Doxapram was administered as an intravenous bolus to pigs and plasma concentration was determined over time. As shown in Table 1, the elimination half-lives of doxapram and ketodoxapram are similar and are in the range of about 2 to 3 hours, with the half-life of the metabolite ketodoxapram tending to be longer.

**Table 1: Pharmacokinetic data for doxapram and its metabolite ketodoxapram in pigs**

| Compound | Pig # | Cₘₐₓ (ng/ml) | Tₘₐₓ (h) | AUC₀₋ₙ (ng/ml*h) | AUC_{0-inf} (ng/ml*h) | AUCₑₓₜᵣₐ (%) | T_{1/2} (h) |
|---|---|---|---|---|---|---|---|
| Doxapram | 98 | 2312 | 0 | 2183 | 2196 | 0.61 | 1.92 |
| | 99 | 5062 | 0 | 3130 | 3179 | 1.53 | 2.65 |
| | 100 | 4903 | 0 | 3473 | 3482 | 0.26 | 1.79 |
| Average | | 4092 | 0 | 2928 | 2952 | 0.80 | 2.12 |
| Ketodoxapram | 98 | 31.78 | 1 | 149.4 | 156.7 | 4.65 | 2.59 |
| | 99 | 63.47 | 1 | 441.7 | 504.0 | 12.36 | 3.48 |
| | 100 | 48.33 | 1 | 254.1 | 264.6 | 3.96 | 2.36 |
| Average | | 47.86 | 1 | 281.7 | 308.4 | 6.99 | 2.81 |

| | | | | | | | |
|---|---|---|---|---|---|---|---|
| AUC₀₋ₙ: Area under the concentration-time curve between administration and last measured data point; AUC_{0-inf}: Total AUC (extrapolated to infinity); AUCₑₓₜᵣₐ: AUC fraction extrapolated to infinity; Cₘₐₓ: Plasma peak concentration; T_{1/2}: plasma half-life; Tₘₐₓ: Time to reach Cₘₐₓ. | | | | | | | |

### e) Pharmacokinetics after long-term administration of doxapram

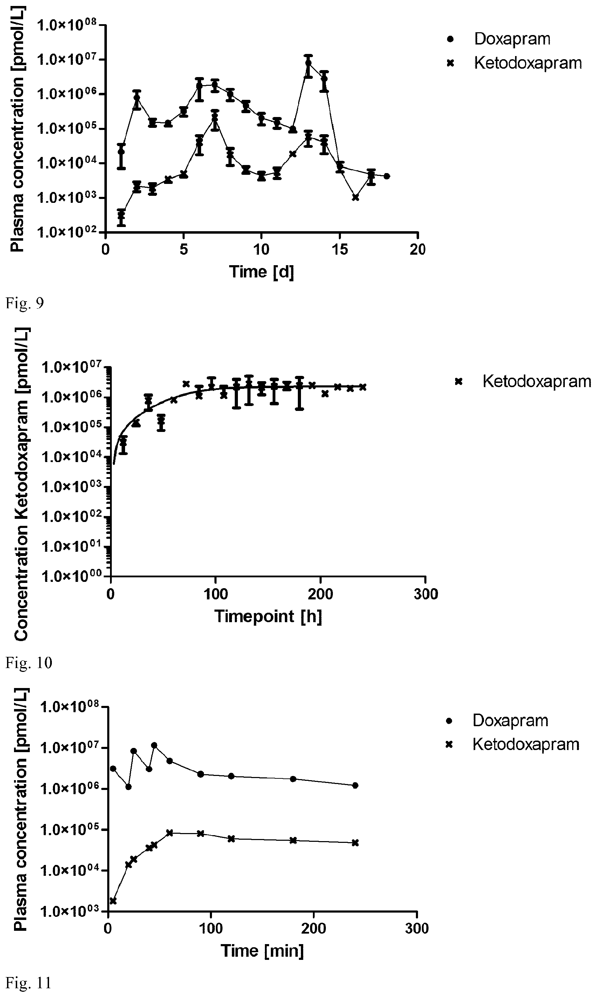

Pigs were administered repeated doses of doxapram over a period of several days. As shown in Fig. 9, there is an initial accumulation phase of doxapram and its metabolite ketodoxapram in the first days, after which the plasma concentrations stabilize. Similarly, after long-term administration of ketodoxapram to two pigs, an accumulation phase of trough concentrations over 4 to 5 days is observed (Fig. 10). Therefore, to reach effective concentrations more rapidly, a treatment start with higher initial doses (loading doses), shorter administration intervals, or a combination thereof could be considered.

### f) Pharmacokinetics of doxapram in a horse

Three intravenous doses of doxapram were administered to a horse. As shown in Fig. 11, the characteristics of porcine doxapram pharmacokinetics are essentially also observed in the horse, including a plasma concentration of ketodoxapram of about 10 % of its parent compound doxapram and an initial metabolite accumulation phase over the first 4-5 days.

Non-standard literature cited:
Bruce et al. (1965), J Med Chem 8:157;
Kirchhof et al. (2016), Eur Heart J 37:2893-962;
Kuck et al. (2016); N Engl J Med 374:2235-45;
Ravens & Odening (2017), Pharmacol Ther 176:13-21;
Ravens et al. (2013), J Physiol 591:4087-97;
Robson & Prescott (1978), Br. J. Pharmac. 7:81;
Schmidt et al. (2011), Vasc Health Risk Manag 7:193-202;
Schmidt et al. (2012), Dtsch Med Wochenschr 137:1654-8;
Schmidt et al. (2014), Eur J Pharmacol 738:250-5;
Schmidt et al. (2015), Circulation 132:82-92;
Schmidt et al. (2017), Eur Heart J 38:1764-74;
Singh et al. (2007), N Engl J Med 357:987-99;
US 2015/0018342 A1;
Voigt & Dobrev (2016); Card Electrophysiol Clin 8:411-21;
WO 2012/166909.

## Claims

1. A compound having a structure as shown in formula (I) or a pharmaceutically acceptable salt thereof, 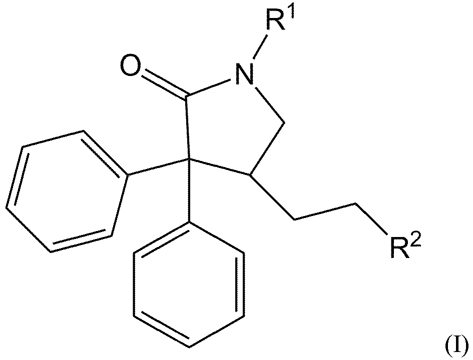 wherein
R¹ is ethyl, -H, or methyl and
R² is a side chain having a structure as shown in formula (II) 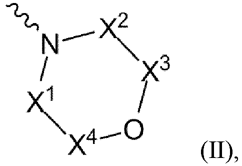 wherein
X¹ is C=O, CH₂, C=S, C-OH, C-NH₂, or C-NO₂,
X² to X⁴ are independently selected from CH₂, C=O, C=S, C-OH, and C-NH₂, or R² is -NH₂,
for use in treating and/or preventing of atrial arrhythmia in a subject.

2. The compound for use of claim 1, wherein R¹ is ethyl and X² to X⁴ are CH₂; and/or wherein X¹ is C=O or CH₂, preferably is C=O.

3. The compound for use of claim 1 or 2, wherein compound of formula (I) has the structure of formula (Ia)

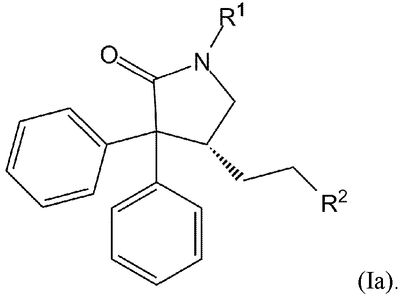

4. The compound for use of any one of claims 1 to 3, wherein said compound has the structure of formula (III), (IV), (V), or (VI)

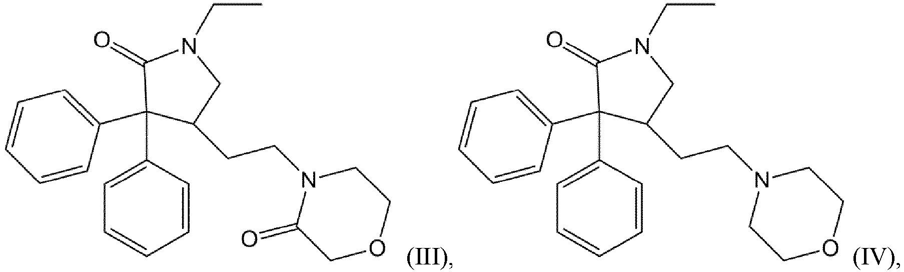

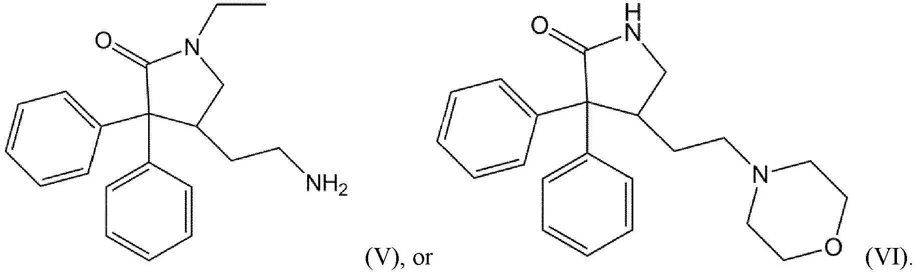

5. The compound for use of any one of claims 1 to 4, wherein said compound is 2-ketodoxapram.

6. The compound for use of any one of claims 1 to 5, wherein said compound is (R)-2-ketodoxapram.

7. The compound for use of any one of claims 1 to 6, wherein said atrial arrhythmia is atrial fibrillation, atrial flutter, premature atrial contraction, supraventricular tachycardias, sick sinus syndrome, and Wolff-Parkinson-White syndrome, preferably is atrial fibrillation.

8. The compound for use of any one of claims 1 to 7, wherein said atrial arrhythmia is new onset atrial fibrillation, paroxysmal atrial fibrillation, persistent atrial fibrillation, or permanent atrial fibrillation.

9. The compound for use of any one of claims 1 to 8, wherein said use comprises avoiding adverse effects caused by central nervous stimulation, in particular epileptic seizures, paresthesias, restlessness, confusion, nausea, vomiting, and/or hallucinations.

10. The compound for use of any one of claims 1 to 9, wherein said use comprises intravenous and/or oral administration.

11. The compound for use of any one of claims 1 to 10, wherein said use comprises intravenous administration of said compound at a dose of from 0.1 mg/kg body weight to 10 mg/kg body weight, preferably of from 0.25 mg/kg body weight to 2 mg/kg body weight, more preferably of from 1.5 mg/kg body weight to 1.8 mg/kg body weight; and/or intravenous administration of said compound at a dose of from 0.004 g/day to 3 g/day, preferably of from 0.01 g/day to 0.3 g/day, more preferably of from 0.05 g/day to 0.27 g/day.

12. The compound for use of any one of claims 1 to 11, wherein said use comprises oral administration of said compound at a dose of from 0.2 mg/kg body weight to 20 mg/kg body weight, preferably of from 0.5 mg/kg body weight to 4 mg/kg body weight, more preferably of from 3 mg/kg body weight to 3.6 mg/kg body weight and/or oral administration of said compound at a dose of from 0.008 g/day to 6 g/day, preferably of from 0.02 g/day to 0.6 g/day, more preferably of from 0.1 g/day to 0.54 g/day.

13. The compound for use of any one of claims 1 to 12, wherein said subject is a mammal, preferably a human.

14. A pharmaceutical composition comprising at least one compound as specified in any one of claims 1 to 4 for use in treating and/or preventing of atrial arrhythmia in a subject.

## Patentansprüche

1. Verbindung mit einer wie in Formel (I) gezeigten Struktur oder pharmazeutisch unbedenkliches Salz davon, 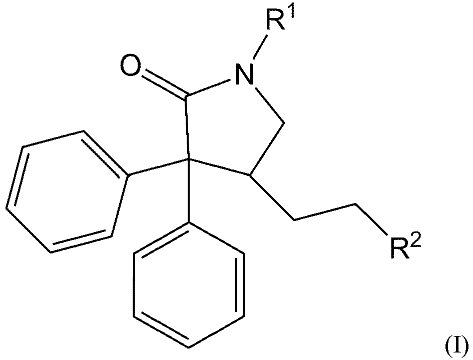 wobei
R¹ für Ethyl, -H oder Methyl steht und
R² für eine Seitenkette mit einer wie in Formel (II) gezeigten Struktur steht 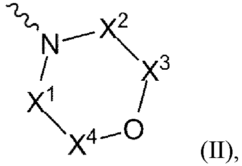 wobei
X¹ für C=O, CH₂, C=S, C-OH, C-NH₂ oder C-NO₂ steht,
X² bis X⁴ unabhängig voneinander aus CH₂, C=O, C=S, C-OH und C-NH₂ ausgewählt sind oder R² für -NH₂ steht,
zur Verwendung bei der Behandlung und/oder Prävention von Vorhofarrhythmie bei einem Subjekt.

2. Verbindung zur Verwendung nach Anspruch 1, wobei R¹ für Ethyl steht und X² bis X⁴ für CH₂ stehen und/oder wobei X¹ für C=O oder CH₂, vorzugsweise C=O, steht.

3. Verbindung zur Verwendung nach Anspruch 1 oder 2, wobei die Verbindung der Formel (I) die Struktur der Formel (Ia) aufweist

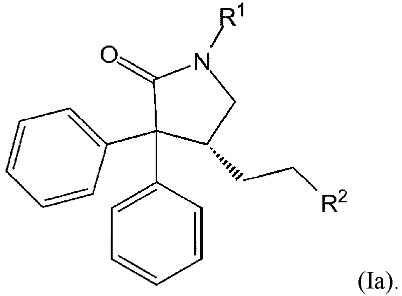

4. Verbindung zur Verwendung nach einem der Ansprüche 1 bis 3, wobei die Verbindung die Struktur der Formel (III), (IV), (V) oder (VI) aufweist

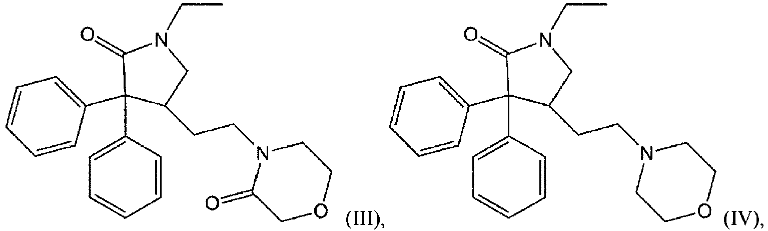

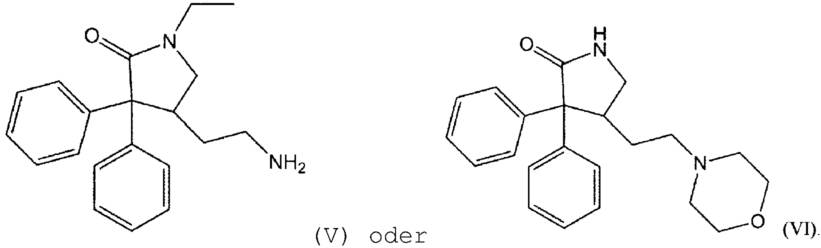

5. Verbindung zur Verwendung nach einem der Ansprüche 1 bis 4, wobei die Verbindung 2-Ketodoxapram ist.

6. Verbindung zur Verwendung nach einem der Ansprüche 1 bis 5, wobei die Verbindung (R)-2-Ketodoxapram ist.

7. Verbindung zur Verwendung nach einem der Ansprüche 1 bis 6, wobei es sich bei der Vorhofarrhythmie um Vorhofflimmern, Vorhofflattern, vorzeitige Vorhofkontraktion, supraventrikuläre Tachykardien, Sick-Sinus-Syndrom und Wolff-Parkinson-White-Syndrom, vorzugsweise Vorhofflimmern, handelt.

8. Verbindung zur Verwendung nach einem der Ansprüche 1 bis 7, wobei es sich bei der Vorhofarrhythmie um neu einsetzendes Vorhofflimmern, paroxysmales Vorhofflimmern, persistierendes Vorhofflimmern oder permanentes Vorhofflimmern handelt.

9. Verbindung zur Verwendung nach einem der Ansprüche 1 bis 8, **dadurch gekennzeichnet, dass** die Verwendung die Vermeidung von Nebenwirkungen durch zentralnervöse Stimulation, insbesondere epileptische Anfälle, Parästhesien, Unruhe, Verwirrtheit, Übelkeit, Erbrechen und/oder Halluzinationen, umfasst.

10. Verbindung zur Verwendung nach einem der Ansprüche 1 bis 9, wobei die Verwendung die intravenöse und/oder orale Verabreichung umfasst.

11. Verbindung zur Verwendung nach einem der Ansprüche 1 bis 10, wobei die Verwendung die intravenöse Verabreichung der Verbindung in einer Dosis von 0,1 mg/kg Körpergewicht bis 10 mg/kg Körpergewicht, vorzugsweise 0,25 mg/kg Körpergewicht bis 2 mg/kg Körpergewicht, besonders bevorzugt 1,5 mg/kg Körpergewicht bis 1,8 mg/kg Körpergewicht und/oder die intravenöse Verabreichung der Verbindung in einer Dosis von 0,004 g/Tag bis 3 g/Tag, vorzugsweise 0,01 g/Tag bis 0,3 g/Tag, besonders bevorzugt 0,05 g/Tag bis 0,27 g/Tag umfasst.

12. Verbindung zur Verwendung nach einem der Ansprüche 1 bis 11, wobei die Verwendung die orale Verabreichung der Verbindung in einer Dosis von 0,2 mg/kg Körpergewicht bis 20 mg/kg Körpergewicht, vorzugsweise 0,5 mg/kg Körpergewicht bis 4 mg/kg Körpergewicht, besonders bevorzugt 3 mg/kg Körpergewicht bis 3,6 mg/kg Körpergewicht und/oder die orale Verabreichung der Verbindung in einer Dosis von 0,008 g/Tag bis 6 g/Tag, vorzugsweise 0,02 g/Tag bis 0,6 g/Tag, besonders bevorzugt 0,1 g/Tag bis 0,54 g/Tag umfasst.

13. Verbindung zur Verwendung nach einem der Ansprüche 1 bis 12, wobei das Subjekt ein Säugetier, vorzugsweise ein Mensch ist.

14. Pharmazeutische Zusammensetzung, umfassend mindestens eine Verbindung nach einem der Ansprüche 1 bis 4 zur Verwendung bei der Behandlung und/oder Prävention von Vorhofarrhythmie bei einem Subjekt.

## Revendications

1. Composé ayant une structure telle que représentée dans la formule (I) ou sel pharmaceutiquement acceptable de celui-ci, 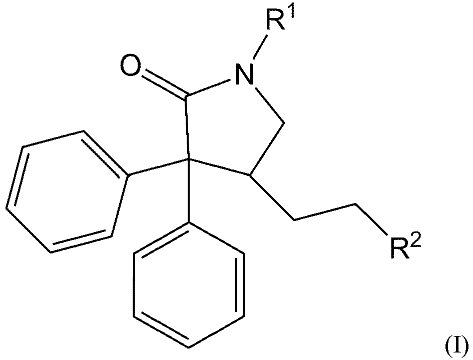 dans laquelle
R¹ est éthyle, -H ou méthyle et
R² est une chaîne latérale ayant une structure telle que représentée dans la formule (II) 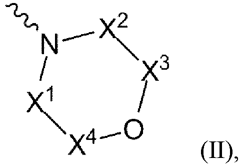
dans laquelle
X¹ est C=O, CH₂, C=S, C-OH, C-NH₂, ou C-NO₂,
X² à X⁴ sont indépendamment choisis parmi CH₂, C=O, C=S, C-OH et C-NH₂, ou R² est -NH₂,
pour une utilisation dans le traitement et/ou la prévention de l'arythmie auriculaire chez un sujet.

2. Composé pour utilisation selon la revendication 1, dans lequel R¹ est éthyle et X² à X⁴ sont CH₂; et/ou dans lequel X¹ est C=O ou CH₂, de préférence C=O.

3. Composé pour utilisation selon la revendication 1 ou 2, dans lequel le composé de formule (I) a la structure de formule (Ia)

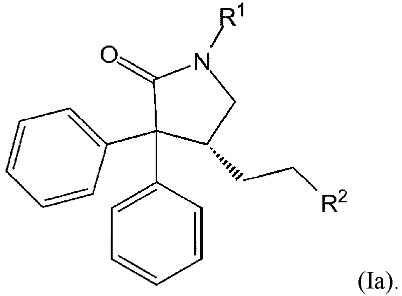

4. Composé pour utilisation selon l'une quelconque des revendications 1 à 3, dans lequel ledit composé a la structure de formule (III), (IV), (V) ou (VI)

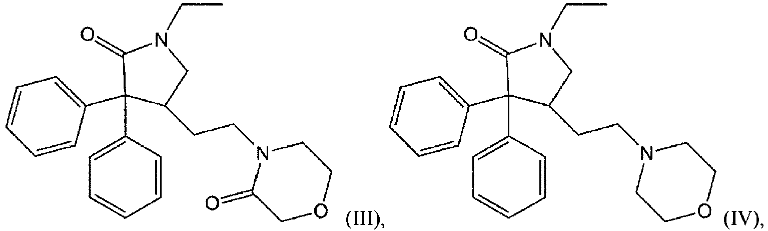

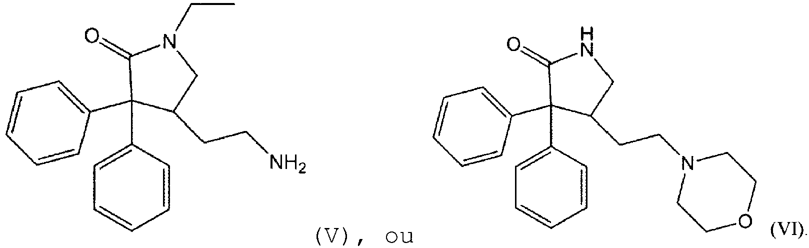

5. Composé pour utilisation selon l'une quelconque des revendications 1 à 4, dans lequel ledit composé est le 2-cétodoxapram.

6. Composé pour utilisation selon l'une quelconque des revendications 1 à 5, dans lequel ledit composé est le (R)-2-cétodoxapram.

7. Composé pour utilisation selon l'une quelconque des revendications 1 à 6, dans lequel ladite arythmie auriculaire est une fibrillation auriculaire, un flutter auriculaire, une contraction auriculaire prématurée, une tachycardie supraventriculaire, un syndrome du sinus malade et un syndrome de Wolff-Parkinson-White, de préférence est une fibrillation auriculaire.

8. Composé pour utilisation selon l'une quelconque des revendications 1 à 7, dans lequel ladite arythmie auriculaire est une fibrillation auriculaire d'apparition nouvelle, une fibrillation auriculaire paroxystique, une fibrillation auriculaire persistante ou une fibrillation auriculaire permanente.

9. Composé pour utilisation selon l'une quelconque des revendications 1 à 8, dans lequel ladite utilisation comprend l'évitement d'effets indésirables provoqués par la stimulation nerveuse centrale, en particulier des crises d'épilepsie, des paresthésies, de l'agitation, de la confusion, des nausées, des vomissements et/ou des hallucinations.

10. Composé pour utilisation selon l'une quelconque des revendications 1 à 9, dans lequel ladite utilisation comprend une administration intraveineuse et/ou orale.

11. Composé pour utilisation selon l'une quelconque des revendications 1 à 10, dans lequel ladite utilisation comprend une administration intraveineuse dudit composé à une dose de 0,1 mg/kg de poids corporel à 10 mg/kg de poids corporel, de préférence de 0,25 mg/kg de poids corporel à 2 mg/kg de poids corporel, plus préférablement de 1,5 mg/kg de poids corporel à 1,8 mg/kg de poids corporel ; et/ou une administration intraveineuse dudit composé à une dose de 0,004 g/jour à 3 g/jour, de préférence de 0,01 g/jour à 0,3 g/jour, plus préférablement de 0,05 g/jour à 0,27 g/jour.

12. Composé pour utilisation selon l'une quelconque des revendications 1 à 11, dans lequel ladite utilisation comprend une administration orale dudit composé à une dose de 0,2 mg/kg de poids corporel à 20 mg/kg de poids corporel, de préférence de 0,5 mg/kg de poids corporel à 4 mg/kg de poids corporel, plus préférablement de 3 mg/kg de poids corporel à 3,6 mg/kg de poids corporel et/ou une administration orale dudit composé à une dose de 0,008 g/jour à 6 g/jour, de préférence de 0,02 g/jour à 0,6 g/jour, plus préférablement de 0,1 g/jour à 0,54 g/jour.

13. Composé pour utilisation selon l'une quelconque des revendications 1 à 12, dans lequel ledit sujet est un mammifère, de préférence un humain.

14. Composition pharmaceutique comprenant au moins un composé tel que spécifié dans l'une quelconque des revendications 1 à 4, destinée à être utilisée dans le traitement et/ou la prévention de l'arythmie auriculaire chez un sujet.
